(19) Europäisches Patentamt
European Patent Office
Office européen des brevets

(11) **EP 4 331 680 A1**

(12) **EUROPEAN PATENT APPLICATION**
published in accordance with Art. 153(4) EPC

(43) Date of publication:
06.03.2024 Bulletin 2024/10

(21) Application number: 22795411.2

(22) Date of filing: 25.03.2022

(51) International Patent Classification (IPC):
*A61P 37/04* (2006.01)        *A61K 31/4172* (2006.01)
*A23L 33/175* (2016.01)

(52) Cooperative Patent Classification (CPC):
A23L 33/175; A61K 31/4172; A61P 37/04

(86) International application number:
PCT/JP2022/014249

(87) International publication number:
WO 2022/230493 (03.11.2022 Gazette 2022/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.04.2021 JP 2021074322

(71) Applicant: Suntory Holdings Limited
Osaka 530-8203 (JP)

(72) Inventors:
• KATSUBE, Makoto
Soraku-gun, Kyoto 619-0284 (JP)
• WATANABE, Hiroshi
Soraku-gun, Kyoto 619-0284 (JP)
• MURAYAMA, Norihito
Soraku-gun, Kyoto 619-0284 (JP)

(74) Representative: Vossius & Partner
Patentanwälte Rechtsanwälte mbB
Siebertstrasse 3
81675 München (DE)

(54) **COMPOSITION FOR INCREASING LEUKOCYTES AND/OR BASOPHILS**

(57)    The present invention aims to provide a composition for increasing leukocytes and/or basophils, a composition for inhibiting epidermal growth factor receptor (EGFR) tyrosine kinase, a composition for activating innate immunity, a method of increasing leukocytes and/or basophils, a method of inhibiting epidermal growth factor receptor (EGFR) tyrosine kinase, and a method of activating innate immunity. The present invention relates to a composition for increasing leukocytes and/or basophils, which contains L-ergothioneine or a salt thereof as an active ingredient.

FIG. 1

**Description**

TECHNICAL FIELD

The present invention relates to a composition for increasing leukocytes and/or basophils. The present invention also relates to a composition for inhibiting epidermal growth factor receptor (EGFR) tyrosine kinase and a composition for activating innate immunity. The present invention further relates to a method of increasing leukocytes and/or basophils, a method of inhibiting epidermal growth factor receptor (EGFR) tyrosine kinase, a method of activating innate immunity, and the like.

BACKGROUND ART

Leukocytes are generally classified into five types: monocytes (macrophages), lymphocytes, neutrophils, basophils, and eosinophils, which are immunocompetent cells involved in biological defense. These cells are hematopoietic stem cell-derived cells that play a role in eliminating foreign substances such as bacteria and viruses that have entered the body from the outside and eliminating tumor cells and cells that have completed their roles. The immune function of these immunocompetent cells is known to decline with aging. Presumedly, the main cause is that the thymus, which is responsible for the production of T cells, and the spleen, which is rich in lymphocytes, atrophy faster than other organs with aging.

Epidermal growth factor receptor (hereinafter also referred to as "EGFR") is a tyrosine kinase receptor that performs signal transduction when a kinase in the cell membrane is activated upon binding of a ligand such as epidermal growth factor (hereinafter also referred to as "EGF") to the receptor. EGFR is mutated or amplified in cancer tissues and known to contribute to canceration of cells, cancer proliferation, invasion, metastasis, and the like. However, EGFR is also expressed in normal tissues and known to prevent virus entry into cells by inhibiting the function of existing EGFR.

Ergothioneine is an amino acid found in mushrooms and the like. L-ergothioneine is present in nature. According to Patent Literature 1, the peritesticular fat weight and the triglyceride (neutral fat) level in plasma were lower in mice orally administered with ergothioneine than in mice not orally administered with ergothioneine.

Patent Literature 2 discloses an agent for promoting the production of immune response activating cytokines, which contains, as active ingredients, ergothioneine and any one Toll-like receptor ligand selected from lipopolysaccharide (LPS), Pam2CSK4, synthetic triacylated lipoprotein, and imidazoquinolines. In the absence of the TLR ligand, none of IL-6, IL-12p40, IL-1$\beta$, and IL-10 was detected in any sample with an ergothioneine concentration of either 0 or 10 mM. In other words, according to the disclosure, when TLR was not stimulated, none of IL-6, IL-12p40, IL-1$\beta$, and IL-10 was detected regardless of the presence or absence of ergothioneine (claim 1 and elsewhere, and paragraph [0068]). Thus, an agent for promoting the production of immune response activating cytokines has been known which contains, as active ingredients, ergothioneine and a specific Toll-like receptor ligand.

CITATION LIST

- Patent Literature

Patent Literature 1: JP 2011-102286 A
Patent Literature 2: JP 6121597 B

SUMMARY OF INVENTION

- Technical Problem

However, it has not been known that L-ergothioneine or a salt thereof has an action to increase leukocytes and/or basophils and an action to inhibit epidermal growth factor receptor (EGFR) tyrosine kinase.

The present invention aims to provide a composition for increasing leukocytes and/or basophils. The present invention also aims to provide a composition for inhibiting epidermal growth factor receptor (EGFR) tyrosine kinase. The present invention also aims to provide a composition for activating innate immunity. The present invention also aims to provide a method of increasing leukocytes and/or basophils, a method of inhibiting epidermal growth factor receptor (EGFR) tyrosine kinase, and a method of activating innate immunity.

- Solution to Problem

As a result of extensive studies to solve the problem described above, the present inventors found that L-ergothioneine has an action to increase leukocytes and/or basophils and an action to inhibit epidermal growth factor receptor (EGFR) tyrosine kinase.

Specifically, the present invention relates to, but is not limited to, a composition for increasing leukocytes and/or basophils and the like described below.

(1) A composition for increasing at least one of leukocytes or basophils, containing L-ergothioneine or a salt thereof as an active ingredient.

(2) A composition for inhibiting epidermal growth factor receptor (EGFR) tyrosine kinase, containing L-ergothioneine or a salt thereof as an active ingredient.

(3) A composition for activating innate immunity, containing the composition according to (1) or (2) above.

(4) The composition according to any one of (1) to (3) above, wherein the composition is an oral composition.

(5) The composition according to any one of (1) to (4) above, wherein the composition is a food or beverage.

(6) The composition according to any one of (1) to (5) above, wherein an amount of L-ergothioneine or a salt thereof in the composition per adult daily intake is 2 to 50 mg in terms of L-ergothioneine.

(7) A method of increasing at least one of leukocytes or basophils, including administering L-ergothioneine or a salt thereof.

(8) A method of inhibiting epidermal growth factor receptor (EGFR) tyrosine kinase, including administering L-ergothioneine or a salt thereof.

(9) A method of activating innate immunity, including administering L-ergothioneine or a salt thereof.

(10) Use of L-ergothioneine or a salt thereof for increasing at least one of leukocytes or basophils.

(11) Use of L-ergothioneine or a salt thereof for inhibiting epidermal growth factor receptor (EGFR) tyrosine kinase.

(12) Use of L-ergothioneine or a salt thereof for activating innate immunity.

- Advantageous Effects of Invention

The present invention can provide a composition for increasing leukocytes and/or basophils. The present invention can also provide a composition for inhibiting epidermal growth factor receptor (EGFR) tyrosine kinase. The present invention can provide a composition for activating innate immunity, a method of increasing leukocytes and/or basophils, a method of inhibiting epidermal growth factor receptor (EGFR) tyrosine kinase, and a method of activating innate immunity.

BRIEF DESCRIPTION OF DRAWINGS

FIG. 1 is a graph showing the amount of change in blood leukocyte count between at the time of pre-testing and at the time of testing on week 4 in a test food group and a control food group.

FIG. 2 is a graph showing the amount of change in blood basophil count between at the time of pre-testing and at the time of testing on week 4 in the test food group and the control food group.

FIG. 3 is a graph showing an activity of L-ergothioneine to inhibit substrate binding of epidermal growth factor receptor (EGFR) tyrosine kinase.

DESCRIPTION OF EMBODIMENTS

The composition for increasing leukocytes and/or basophils of the present invention contains L-ergothioneine or a salt thereof as an active ingredient. Hereinafter, the composition for increasing leukocytes and/or basophils of the present invention is sometimes referred to as "the composition according to the first embodiment of the present invention".

The composition for inhibiting epidermal growth factor receptor (EGFR) tyrosine kinase of the present invention contains L-ergothioneine or a salt thereof as an active ingredient. Hereinafter, the composition for inhibiting epidermal growth factor receptor (EGFR) tyrosine kinase of the present invention is sometimes referred to as "the composition according to the second embodiment of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula edodes*), hen-of-the-wood (binomial name: *Grifola Frondosa*), reishi mushroom (binomial name: *Ganoderma lucidum*), lion's mane mushroom (binomial name: *Hericium erinaceus*), Yanagi-matsutake (binomial name: *Agrocybe aegerita*), girolle (binomial name: *Cantharellus cibarius*), porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

As shown in Examples (described later), ingestion of L-ergothioneine significantly increased the blood leukocyte count and basophil count, compared to no ingestion.

Thus, L-ergothioneine can be used as an active ingredient for promoting an increase in blood leukocytes and/or basophils. The blood leukocyte count and basophil count can be measured by known methods. Herein, the blood leukocytes include the following five types: monocytes (macrophages), lymphocytes, neutrophils, basophils, and eosinophils. The blood leukocyte count is the total number of these.

Having an action to increase blood leukocytes and basophils, L-ergothioneine or a salt thereof has an action to activate innate immunity.

The action to increase blood leukocytes may be achieved by any means such as moderating or reducing a decline in leukocyte count or promoting the recovery of leukocyte count. The action refers to an achievement of an effect of increasing blood leukocytes by ingestion of L-ergothioneine or a salt thereof.

The action to increase blood basophils may be achieved by any means such as moderating or reducing a decline in basophil count or promoting the recovery of basophil count. The action refers to an achievement of an effect of increasing blood basophils by ingestion of L-ergothioneine or a salt thereof.

As shown in Examples (described later), L-ergothioneine has an activity to inhibit epidermal growth factor receptor (EGFR) tyrosine kinase.

Thus, L-ergothioneine can be used as an active ingredient for inhibiting epidermal growth factor receptor (EGFR) tyrosine kinase. The activity to inhibit epidermal growth factor receptor (EGFR) tyrosine kinase can be measured by a known method. For example, as shown in Examples (described later), the activity can be measured with an assay system using insect cells.

Having an action to inhibit epidermal growth factor receptor (EGFR) tyrosine kinase, L-ergothioneine or a salt thereof has an action to activate innate immunity. L-ergothioneine or a salt thereof can be used as an active ingredient for activating innate immunity.

The expression "activity to inhibit epidermal growth factor receptor (EGFR) tyrosine kinase" encompasses selective binding of a ligand to EGFR and no action being expressed by EGFR upon binding (i.e., EGFR is not activated).

The composition according to the first embodiment and/or the composition according to the second embodiment of the present invention can be used in a composition for activating innate immunity. Thus, a composition for activating innate immunity which contains the composition according to the first embodiment and/or the composition according to the second embodiment of the present invention is also encompassed by the present invention. In one embodiment, the composition according to the first embodiment of the present invention can be used to activate innate immunity. The composition according to the second embodiment of the present invention can be used to activate innate immunity.

The composition according to the first embodiment of the present invention, the composition according to the second embodiment of the present invention, and the composition for activating innate immunity of the present invention (also referred to as "the composition according to the third embodiment of the present invention") each have an action for activating innate immunity and can be used to prevent or ameliorate a condition or disease caused by a decline in innate immunity.

Preventing a condition or disease encompasses preventing the onset, delaying the onset, reducing the incidence rate, reducing the onset risk, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

Examples of the condition or disease caused by a decline in innate immunity include infectious diseases caused by microorganisms such as viruses and bacteria and various malignant tumors. Examples of the infectious diseases include infectious enteritis caused by bacteria such as cholera, enterotoxigenic Escherichia coli, Shigella, and Salmonella, and viruses through oral infection; influenza and common cold syndromes through respiratory tract infection; and stomatitis and periodontal disease through intraoral infection. Examples of the malignant tumors include epithelial malignant tumors that occur in solid organs such as the gastrointestinal tract, respiratory mucosa, liver, and kidney; and non-epithelial malignant tumors that occur in locomotor organs, soft tissues, and the like. Examples of the infectious diseases also include symptoms from a virus or the like, such as herpes virus, with which a subject has been infected in the past and which has been dormant in the body of the subject.

Hereinafter, the composition for increasing leukocytes and/or basophils of the present invention, the composition for inhibiting epidermal growth factor receptor (EGFR) tyrosine kinase of the present invention, and the composition for activating innate immunity of the present invention are sometimes simply referred to as "the composition of the present invention" as a collective term. Preferably, the composition of the present invention does not contain Toll-like receptor ligands.

The composition of the present invention is applicable for therapeutic use (medical use) and non-therapeutic use (non-medical use). The term "non-therapeutic" is a concept that does not include medical activities, i.e., a concept that does not include methods of surgery, therapy, or diagnosis of humans.

The composition of the present invention can be provided as an agent or the like, for example, but it is not limited thereto. The agent can be directly provided as a composition or can be provided as a composition containing the agent. In one embodiment, the composition for increasing leukocytes and/or basophils of the present invention can also be referred to as an agent for increasing leukocytes and/or basophils. In one embodiment, the composition for inhibiting epidermal growth factor receptor (EGFR) tyrosine kinase of the present invention can also be referred to as an agent for inhibiting epidermal growth factor receptor (EGFR) tyrosine kinase. In one embodiment, the composition for activating innate immunity of the present invention can also be referred to as an agent for activating innate immunity.

In order to sufficiently obtain the effect of the present invention, preferably, the composition of the present invention is an oral composition. The oral composition may be a food or beverage, an oral pharmaceutical product, an oral quasi-pharmaceutical product, or feed, preferably a food or beverage or an oral pharmaceutical product, more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in food and beverages, pharmaceutical products, quasi-pharmaceutical products, feed, and the like can be used. Preferably, the composition of the present invention does not contain Toll-like receptor ligands. When the composition of the present invention is provided as a food or beverage, a pharmaceutical product, a quasi-pharmaceutical product, feed, or the like, the production method is not limited, and any common method can be used for production.

For example, when the composition of the present invention is provided as a food or beverage, various types of food or beverages can be provided by adding a component usable in food and beverages (e.g., a food material or an optional food additive) to L-ergothioneine or a salt thereof. Non-limiting examples of the food or beverage include general food and beverages, health foods, health beverages, foods with function claims, foods for specified health uses, dietary supplements, and food and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition of the present invention is not limited and can be set according to the composition form and the like.

The amount of L-ergothioneine or a salt thereof in the composition of the present invention is, for example, preferably 0.0001 wt% or more, more preferably 0.001 wt% or more and is preferably 90 wt% or less, more preferably 50 wt% or less in terms of L-ergothioneine. In one embodiment, the amount of L-ergothioneine or a salt thereof in the composition is preferably 0.0001 to 90 wt%, more preferably 0.001 to 50 wt% in terms of L-ergothioneine. In one embodiment, when the composition of the present invention is provided as a food or beverage, a pharmaceutical product, a quasi-pharmaceutical product, feed, or the like, preferably, the amount of L-ergothioneine or a salt thereof is in the above ranges.

In the case of L-ergothioneine, the expression "the amount in terms of ergothioneine" or a similar expression refers to the amount of L-ergothioneine. In the case of a salt of L-ergothioneine, the expression refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an effect of increasing leukocytes and/or basophils, an effect of inhibiting epidermal growth factor receptor (EGFR) tyrosine kinase, or an effect of activating innate immunity. The intake may be appropriately set according to the administration form, administration method, body weight of a subject, and the like.

In one embodiment, when orally feeding or administering the composition of the present invention to a human (adult) as a subject, the intake of L-ergothioneine or a salt thereof is preferably 2 mg or more, more preferably 5 mg or more, still more preferably 10 mg or more, and is preferably 50 mg or less, more preferably 25 mg or less, still more preferably 20 mg or less in terms of L-ergothioneine per day. In one embodiment, when orally feeding or administering to a human (adult) as a subject, the intake of L-ergothioneine or a salt thereof is preferably 2 to 50 mg, more preferably 5 to 25 mg, still more preferably 5 to 20 mg, particularly preferably 10 to 20 mg in terms of L-ergothioneine per day. Preferably, the above amount of the composition is fed or administered in one or more portions per day, for example, in one portion or several portions (for example, two or three portions) per day. In one embodiment of the present invention, the composition of the present invention may be an oral composition for feeding or administering a human with the above amount of L-ergothioneine or a salt thereof per 60 kg body weight per day.

In one embodiment, when parenterally administering the composition of the present invention to a human (adult), the administration amount of L-ergothioneine or a salt thereof is, for example, preferably 2 to 50 mg, more preferably 5 to 25 mg, still more preferably 5 to 20 mg, particularly preferably 10 to 20 mg in terms of L-ergothioneine per day.

In one embodiment, in the case of a human (adult), preferably, the above amount of L-ergothioneine or a salt thereof is fed or administered per 60 kg body weight per day.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is expected to result in a higher effect of increasing leukocytes and/or basophils. Continuous feeding or administration of L-ergothioneine or a salt thereof is expected to result in a higher effect of inhibiting epidermal growth factor receptor (EGFR) tyrosine kinase. Continuous feeding or administration of L-ergothioneine or a salt thereof is expected to result in a higher effect of activating innate immunity.

Thus, in a preferred embodiment, the composition of the present invention is continuously fed or administered. In one embodiment of the present invention, the composition of the present invention is continuously fed or administered preferably for at least one week, more preferably for at least two weeks.

The composition of the present invention may be fed or administered to any subject (subject to administration). The subject is preferably a human or non-human mammal, more preferably a human.

The composition of the present invention may be fed or administered, for example, to a subject needing or wanting to increase leukocytes and/or basophils, a subject needing or wanting to inhibit epidermal growth factor receptor (EGFR) tyrosine kinase, and a subject needing or wanting to activate innate immunity. In one embodiment, examples of the subject to administration of the present invention include subjects with a decline in innate immunity and patients with any of the above-described conditions or diseases caused by a decline in innate immunity. In one embodiment, the subject to administration of the composition of the present invention may be a healthy person. The subject to administration of the composition of the present invention may also be a healthy person exhibiting an aging-related decline in immune function of immunocompetent cells.

The composition of the present invention may be labeled with a function claim based on the increase in leukocytes and/or basophils, a function claim based on the inhibition of epidermal growth factor receptor (EGFR) tyrosine kinase, and a function claim based on the activation of innate immunity. For example, the composition of the present invention may be labeled with one or more function claims such as "increasing leukocytes", "increasing basophils", "inhibiting epidermal growth factor receptor (EGFR) tyrosine kinase", and "activating innate immunity".

In one embodiment of the present invention, preferably, the composition of the present invention is a food or beverage labeled with such a function claim. The label may be one indicating use of the composition to obtain the function. The label may be directly attached to the composition or may be attached to a container or a package of the composition.

The present invention encompasses the following methods and uses:

a method of increasing at least one of leukocytes or basophils, including feeding or administering L-ergothioneine or a salt thereof;
a method of inhibiting epidermal growth factor receptor (EGFR) tyrosine kinase, including feeding or administering L-ergothioneine or a salt thereof;
a method of activating innate immunity, including feeding or administering L-ergothioneine or a salt thereof;
use of L-ergothioneine or a salt thereof to increase at least one of leukocytes or basophils;
use of L-ergothioneine or a salt thereof to inhibit epidermal growth factor receptor (EGFR) tyrosine kinase; and
use of L-ergothioneine or a salt thereof to activate innate immunity.

Administering or feeding L-ergothioneine or a salt thereof to a subject can increase the leukocyte count and/or basophil count and can also inhibit epidermal growth factor receptor (EGFR) tyrosine kinase, thus achieving the effect of activating innate immunity and the like. Preferably, L-ergothioneine or a salt thereof is orally administered or fed.

The methods may be therapeutic or non-therapeutic. The uses may be therapeutic or non-therapeutic.

In the methods and uses, L-ergothioneine or a salt thereof, preferred embodiments thereof, and the like are as described above for the composition of the present invention. In the methods and uses, preferably, L-ergothioneine or a salt thereof is fed or administered to a subject one or more times per day, for example, one to several times (e.g., two to three times) per day. The use is preferably for humans or non-human mammals, more preferably for humans. In one embodiment, L-ergothioneine or a salt thereof can be used to provide the effect of activating innate immunity by increasing leukocytes and/or basophils or by inhibiting epidermal growth factor receptor (EGFR) tyrosine kinase. In one embodiment, the method of increasing leukocytes and/or basophils can be used to activate innate immunity. In one embodiment, the method of inhibiting epidermal growth factor receptor (EGFR) tyrosine kinase can be used to activate innate immunity.

0 0 3 9 1 In the methods and uses, L-ergothioneine or a salt thereof is simply used in an amount (effective amount) that provides the effect of increasing leukocytes and/or basophils.

In the methods and uses, L-ergothioneine or a salt thereof is simply used in an amount (effective amount) that provides the effect of inhibiting epidermal growth factor receptor (EGFR) tyrosine kinase.

In the methods and uses, L-ergothioneine or a salt thereof is simply used in an amount (effective amount) that provides the effect of activating innate immunity.

L-ergothioneine or a salt thereof, preferred administration amounts thereof, preferred administration subjects, and the like are as described above for the composition of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, feed, or the like for use in increasing leukocytes and/or basophils. In one embodiment, the present invention also encompasses use of L-ergothioneine or a salt thereof to produce a composition for increasing leukocytes and/or basophils.

The present invention also encompasses L-ergothioneine or a salt thereof for use in increasing leukocytes and/or basophils.

L-ergothioneine or a salt thereof can be used to produce a food or beverage, a pharmaceutical product, a quasi-pharmaceutical product, feed, or the like for use in inhibiting epidermal growth factor receptor (EGFR) tyrosine kinase. In one embodiment, the present invention also encompasses use of L-ergothioneine or a salt thereof to produce a composition for inhibiting epidermal growth factor receptor (EGFR) tyrosine kinase.

The present invention also encompasses L-ergothioneine or a salt thereof for use in inhibiting epidermal growth factor receptor (EGFR) tyrosine kinase.

L-ergothioneine or a salt thereof can also be used to produce a food or beverage, a pharmaceutical product, a quasi-pharmaceutical product, feed, or the like for use in activating innate immunity. In one embodiment, the present invention also encompasses use of L-ergothioneine or a salt thereof to produce a composition for activating innate immunity.

The present invention also encompasses L-ergothioneine or a salt thereof for use in activating innate immunity.

EXAMPLES

The present invention is described in further detail below with reference to examples, but the scope of the present invention is not limited thereto.

<Example 1>

(Study on evaluation of blood leukocyte count and basophil count in humans)

In order to evaluate the influence of supplements containing ergothioneine on blood leukocyte count and basophil count in humans, a placebo-controlled, randomized, double-blind, parallel-group study was conducted on subjects including male and female adults (46 subjects in a test food group; 46 subjects in a control food group; 92 subjects in total). In this study, one capsule containing 20 mg ergothioneine (test food) or one capsule not containing ergothioneine (control food) was fed per day for four weeks. The blood leukocyte count and basophil count in each subject were measured for pre-testing before starting the study (before starting ingestion of the test food or control food). After ingestion of the test food or control food for four weeks, the blood leukocyte count and basophil count in each subject were measured again (testing on week 4).

(Food for evaluation)

Two types of food for evaluation, which were indistinguishable in appearance, flavor, and the like, were used.

• Test food: capsule containing a test substance (20 mg L-ergothioneine)
• Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

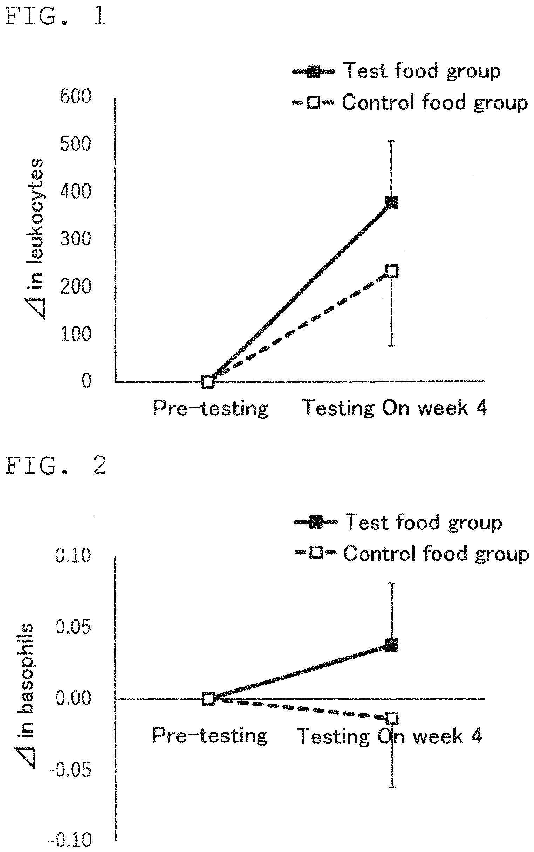

FIG. 1 shows the amount of change in blood leukocyte count in the test food group and the control food group. FIG. 2 shows the amount of change in basophil count in the test food group and the control food group. In FIG. 1 and FIG. 2, ■ (black square) indicates the test food group, and □ (white square) indicates the control food group.

FIG. 1 shows the amount of change in blood leukocyte count ($\Delta$ in leukocytes (cells/$\mu$L)) between at the time of pre-testing and at the time of testing on week 4 in the test food group and the control food group. The amount of change in blood leukocyte count (cells/$\mu$L) shown in FIG. 1 is the amount of change relative to the measurements at the time of pre-testing. Specifically, the amount of change in the test food group at the time of testing on week 4 was determined for the test food group by subtracting the blood leukocyte count (average) at the time of pre-testing from the blood leukocyte count (average) at the time of testing on week 4. Similarly, the amount of change at the time of testing on week 4 was determined for the control food group by subtracting the blood leukocyte count (average) at the time of pre-testing from the blood leukocyte count (average) at the time of testing on week 4. The amount of change at the time of pre-testing was set to 0.

According to the results, the blood leukocytes were increased more in the test food group than in the control food group.

FIG. 2 shows the amount of change in blood basophil count ($\Delta$ in basophils (%)) between at the time of pre-testing and the time of testing on week 4 in the test food group and the control food group. For each group, $\Delta$ in basophils (%) was determined from the blood basophil count (average) using the following formula.

```
Δ in basophils (%) = 100 × ((blood basophil count at the
time of testing on week 4) - ((blood basophil count at the
time of pre-testing))/(blood basophil count at the time of
pre-testing)
```

According to the results, the blood basophils were increased more in the test food group than in the control food group.

<Example 2>

(Evaluation of activity to inhibit substrate binding of epidermal growth factor receptor (EGFR) tyrosine kinase)

The study was commissioned to Eurofins Panlabs. The study was performed using recombinant human epidermal growth factor receptor (EGFR) tyrosine kinase expressed in insect cells. First, epidermal growth factor receptor (EGFR) tyrosine kinase (0.134 $\mu$g/mL) was incubated in HEPES buffer (pH 7.4) with 100 or 1000 $\mu$M L-ergothioneine at 37°C for 15 minutes. Then, 0.2 mg/mL Poly(Glu:Tyr), 10 $\mu$M ATP, and 0.25 $\mu$Ci [$\gamma^{32}$P] ATP were added. Thereby, an enzymatic reaction was initiated. After incubating at 37°C for 30 minutes, 3% $H_3PO_4$ was added to terminate the reaction. Lastly, the activity of ergothioneine to inhibit substrate binding was evaluated using the amount of [$^{32}$P] Poly(Glu:Tyr) formed in the reaction as an index.

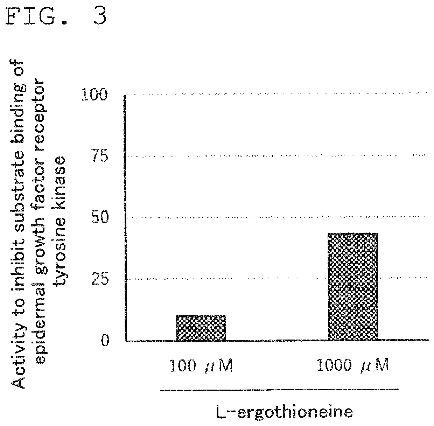

FIG. 3 shows the results of evaluation of the activity of L-ergothioneine to inhibit substrate binding of epidermal growth factor receptor (EGFR) tyrosine kinase. The concentration ($\mu$M) on the horizontal axis is the concentration of L-ergothioneine. L-ergothioneine showed its activity in a concentration-dependent manner and showed an activity to inhibit substrate binding of epidermal growth factor receptor (EGFR) tyrosine kinase. L-ergothioneine was found to have an activity to inhibit epidermal growth factor receptor (EGFR) tyrosine kinase.

**Claims**

1. A composition for increasing at least one of leukocytes or basophils, comprising
   L-ergothioneine or a salt thereof as an active ingredient.

2. A composition for inhibiting epidermal growth factor receptor (EGFR) tyrosine kinase, comprising
   L-ergothioneine or a salt thereof as an active ingredient.

3. A composition for activating innate immunity, comprising
   the composition according to claim 1 or 2.

4. The composition according to any one of claims 1 to 3,
   wherein the composition is an oral composition.

5. The composition according to any one of claims 1 to 4,
   wherein the composition is a food or beverage.

6. The composition according to any one of claims 1 to 5,
   wherein an amount of L-ergothioneine or a salt thereof in the composition per adult daily intake is 2 to 50 mg in terms of L-ergothioneine.

7. A method of increasing at least one of leukocytes or basophils, comprising
   administering L-ergothioneine or a salt thereof.

8. A method of inhibiting epidermal growth factor receptor (EGFR) tyrosine kinase, comprising
   administering L-ergothioneine or a salt thereof.

9. A method of activating innate immunity, comprising
   administering L-ergothioneine or a salt thereof.

10. Use of L-ergothioneine or a salt thereof for increasing at least one of leukocytes or basophils.

11. Use of L-ergothioneine or a salt thereof for inhibiting epidermal growth factor receptor (EGFR) tyrosine kinase.

12. Use of L-ergothioneine or a salt thereof for activating innate immunity.

FIG. 1

FIG. 2

FIG. 3

## INTERNATIONAL SEARCH REPORT

| International application No. |
| --- |
| **PCT/JP2022/014249** |

| A. | CLASSIFICATION OF SUBJECT MATTER |
| --- | --- |

*A61P 37/04*(2006.01)i; *A61K 31/4172*(2006.01)i; *A23L 33/175*(2016.01)i
FI:   A23L33/175; A61K31/4172; A61P37/04

According to International Patent Classification (IPC) or to both national classification and IPC

| B. | FIELDS SEARCHED |
| --- | --- |

Minimum documentation searched (classification system followed by classification symbols)

A61P37/04; A61K31/4172; A23L33/175

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2017-218431 A (THREE B CO LTD) 14 December 2017 (2017-12-14) | 1-12 |
| | claims 5, 10, paragraphs [0027], [0059] | |
| A | paragraph [0002] | 1-12 |
| X | JP 2021-23256 A (NAGASE & CO LTD) 22 February 2021 (2021-02-22) | 1-12 |
| | claims 1, 3, paragraphs [0031], [0060] | |
| X | JP 2012-180329 A (HOKKAIDO UNIV) 20 September 2012 (2012-09-20) | 1-12 |
| | claim 1, paragraphs [0040], [0044] | |
| A | claims 1-5 | 1-12 |
| X | WO 2006/038527 A1 (THREE B CO LTD) 13 April 2006 (2006-04-13) | 1-12 |
| | claims 1-10 | |
| X | 増田　潤子　ほか, 抗酸化物質エルゴチオネインの免疫細胞を介した腫瘍抑制効果への期待, 月刊アグリバイオ, 2020, 4(13), pp. 91-93 in particular, p. 93, Conclusion, (MASUDA, Junko et al. Expectations for the immune cell-mediated tumor suppressive effect of the antioxidant ergothioneine. Agricultural biotechnology.) | 1-12 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 June 2022** | **14 June 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
**Information on patent family members**

International application No.

**PCT/JP2022/014249**

| Patent document cited in search report | | | Publication date (day/month/year) | Patent family member(s) | | | Publication date (day/month/year) |
|---|---|---|---|---|---|---|---|
| JP | 2017-218431 | A | 14 December 2017 | JP | 6121597 | B1 | |
| JP | 2021-23256 | A | 22 February 2021 | (Family: none) | | | |
| JP | 2012-180329 | A | 20 September 2012 | (Family: none) | | | |
| WO | 2006/038527 | A1 | 13 April 2006 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011102286 A
- JP 6121597 B